(19) **Europäisches Patentamt**
**European Patent Office**
**Office européen des brevets**

(11) **EP 3 295 167 B1**

(12) **EUROPEAN PATENT SPECIFICATION**

(45) Date of publication and mention
of the grant of the patent:
**27.02.2019 Bulletin 2019/09**

(21) Application number: **16725061.2**

(22) Date of filing: **13.05.2016**

(51) Int Cl.:
**G01N 33/15** *(2006.01)*     **G01N 13/00** *(2006.01)*

(86) International application number:
**PCT/EP2016/060823**

(87) International publication number:
**WO 2016/184800 (24.11.2016 Gazette 2016/47)**

(54) **METHOD FOR DETERMINING RELEASE RATES OF ACTIVE PRINCIPLE INGREDIENTS FROM AT LEAST ONE SEMISOLID FORM**

VERFAHREN ZUR BESTIMMUNG DER FREISETZUNGSRATEN VON AKTIVPRINZIPWIRKSTOFFEN AUS MINDESTENS EINER HALBFESTEN FORM

PROCÉDÉ PERMETTANT DE DÉTERMINER LES VITESSES DE LIBÉRATION DES INGRÉDIENTS À PRINCIPE ACTIF À PARTIR D'AU MOINS UNE  FORME SEMI-SOLIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2015 EP 15305734**

(43) Date of publication of application:
**21.03.2018 Bulletin 2018/12**

(73) Proprietor: **Galderma Research & Development 06410 Biot (FR)**

(72) Inventors:
• **ALAIN, Brzokewicz**
  **06560 Valbonne (FR)**
• **HUGUET, Hélène**
  **F-06210 Mandelieu La Napoule (FR)**

(74) Representative: **Cabinet Becker et Associés**
  **25, rue Louis le Grand**
  **75002 Paris (FR)**

(56) References cited:
**WO-A1-2005/095950**

• **DEEPIKA ARORA ET AL: "In Vitro Aqueous Fluid-Capacity-Limited Dissolution Testing of Respirable Aerosol Drug Particles Generated from Inhaler Products", PHARMACEUTICAL RESEARCH, KLUWER ACADEMIC PUBLISHERS-PLENUM PUBLISHERS, NL, vol. 27, no. 5, 13 March 2010 (2010-03-13), pages 786-795, XP019793953, ISSN: 1573-904X, DOI: 10.1007/S11095-010-0070-5**
• **Clarence T Ueda ET AL: "Topical and Transdermal Drug Products The Topical/Transdermal Ad Hoc Advisory Panel for the USP Performance Tests of Topical and Transdermal Dosage Forms", , 1 May 2009 (2009-05-01), XP055292519, Retrieved from the Internet: URL:http://www.usp.org/sites/default/files /usp_pdf/EN/USPNF/transdermalStimArticle.p df [retrieved on 2016-08-01]**

**Description**

The invention relates to a method for determining *in vitro* release rate of at least one active principle ingredient from at least one semisolid form.

**Background of the invention**

Dissolution testing in the pharmaceutical industry has been employed as fundamental tool in the formulation design and quality control of finished products. Although initially employed for solid oral dosage forms it has widened its concept and application for semisolid dosage forms to be applied topically on the skin. In this special dosage forms the test is usually referred and named as *in vitro* release testing (IVRT), since the drug in dissolved state in the vehicle has to diffuse and be released by the vehicle, becoming available to penetrate into skin.

In May 1997, FDA released a guideline entitled Scale-up and Post Approval Changes: Chemistry, Manufacturing and Controls, In Vitro Release Testing and In-vivo Bioequivalence Documentation for Non Sterile Semisolid Dosage Form (SUPAC-SS). The guideline is dedicated to semisolid forms, such as creams, gels, lotions, and ointments, intended for topical routes of administration. The vehicle composition and design strongly influence the product performance and how rapid the drug will then be released into the skin. An *in vitro* release rate can reflect the combined effect of several physical and chemical parameters, including solubility and particle size of the active principle ingredient (API), and rheological properties of the dosage form.

Consequently, IVRT offer the possibility to avoid *in-vivo* bioequivalence testing, for changes until Level 2 (changes which could have a significant impact on formulation quality and performance of the product). The IVRT can answer to the following issues: the formulation may undergo postapproval changes and thus need approvals from regulatory affairs, do changes envisioned for the formulation influence the release performance, assessment of the sameness between batches produced for instance in different manufacturing sites, having predictive estimates in respect to the *in-vivo* performance of a drug product before proceeding to biopharmaceutical characterization, having a cost-saving formulation screening, reducing the number of candidates for the next development phases.

*In vitro* release of API from topical and transdermal products, and subsequent permeation through a membrane, can be tested in a vertical diffusion cell (i.e. Franz diffusion cell). In this apparatus, formulation is applied or put in contact with a membrane that is in contact with a receiving medium. The receiving medium is sampled as a function of time and API is quantitated to determine a permeation/flux profile. Membrane materials include synthetic polymer, cadaver or animal skin, and tissue constructs. The choice of membrane is driven by the purpose of the test (i.e. development vs. quality control) and robustness of the model. This technique is applicable not only to externally applied topical formulations, but also to products that deliver via the vaginal, rectal, buccal, or nasal routes. The membrane separates the donor compartment containing the test product from the receptor compartment filled with collection medium. Diffusion of the API from the semisolid product across the membrane is monitored by assay of sequentially collected samples of the receptor medium. At predetermined time points, an aliquot of medium is removed from the receptor compartment for drug content analysis, usually by HPLC. The receptor compartment is topped off with fresh medium after each sampling.

The Franz diffusion cell requires a manual positioning or assembly of the donor compartment into the receptor compartment with the membrane separating both compartments. Because of the membrane which is usually thin for IVRT (generally, a thickness from 30 $\mu$m to 200 $\mu$m), this handling is difficult and time consuming; it may end up with a sealing between compartments unsatisfactory giving rise to unreliable results.

The Applicant recently discovered that replacing Franz diffusion cells by microplates renders IVRT very easy to carry out, while achieving reliable results.

WO2005/095950 discloses the use of micro-titre multi-well plates for evaluating pharmaceutical compositions such as creams by first diluting the composition by introducing it into the donor compartment side with a feed solution.

**Summary of the invention**

The present invention describes a method for determining *in vitro* release rate of at least one active principle ingredient from at least one semisolid form, using a composite multi-well microtitre plate, wherein each well comprises a donor compartment and a corresponding acceptor compartment adapted to receive the donor compartment, the bottom of the donor compartment is made of a porous membrane, the method comprising:

> a. Placing an aliquot of a semisolid form comprising at least one active principle ingredient in at least one donor compartment of the multi-well microtitre plate;
> b. Placing an initial acceptor medium in the corresponding acceptor compartment(s);
> c. Inserting the donor compartment in the corresponding acceptor compartment, so that the membrane in the bottom of the donor compartment is into contact with the initial acceptor medium;

d. Collecting at at least two successive time intervals a sample of the acceptor medium in the acceptor compartment;
e. Measuring the release rate of said active principle ingredient in each collected samples.

Accordingly, the present invention presents several advantages: the wells can have standard sizes, can be disposable, and, as a multi-well microtitre plate, it is very easy to handle at a high screen rate. The present invention provides a cost and time saving method for determining release rates of active principle ingredient from a semisolid dosage form.

In accordance with the present invention, the at least one semi-solid form is a cream, gel, lotion or ointment, intended for topical routes of administration. The semisolid forms are more particularly forms that are topically applied onto the skin or its appendages (such as nails or hair).

## Brief Description of the Figures

Figure 1: Picture of a composite 24-well microtitre plate (multi-well microtitre receiver -or donor- plate and multi-well microtitre reservoir -or acceptor- plate) with a scheme of one of its wells comprising a donor compartment with a formulation (50-250mg) and an acceptor compartment, the bottom of the donor compartment is made of a synthetic membrane, and the receptor (or acceptor) medium (600 $\mu$l) is in the receptor compartment.

Figure 2: Fig. 2A: Dosage strength: Amount of metronidazole released ($\mu$g/cm$^2$) by square root of time (h$^{1/2}$) with different concentrations of metronidazole in creams. Fig. 2B: Viscosity: % API released by viscosity (Cps) of metronidazole containing gels. Figure 3: Fig. 3A: % API released by time (h) with different amounts of applied formulation (metronidazole containing cream). Fig. 3B: Flux (g/cm$^2$/h$^{1/2}$) by amount applied (mg).

Figure 4: Fig. 4A: Amount of API released ($\mu$g/cm$^2$) by square root of time (h$^{1/2}$) with IVRT Franz cells. Fig. 4B: Amount of API released ($\mu$g/cm$^2$) by square root of time (h$^{1/2}$) with IVRT microplate according to the invention.

Figure 5: Scheme of the runs made with IVRT Franz cells or IVRT Microplates. Wells are represented with two different semisolid forms (R is the reference one and T is the tested one).

Figure 6: T/R ratio values by number of T/R ratio ($\Delta$: 24-well microplate according to the invention; ■ Franz cells)

Figure 7: Automation: Amount of metronidazole released ($\mu$g/cm$^2$) by square root of time (h$^{1/2}$) with different 0.5 and 0.75 % metronidazole creams.

## Detailed description of the invention:

The present invention therefore aims at determining *in vitro* release rate of at least one active principle ingredient from at least one semisolid form.

The active principle ingredient (or API, or drug) can be any compound that has an effective biological effect on a subject (i.e., a human and/or non-human mammal), particularly on the skin or its appendages (such as nails or hair). The active ingredient can be one ingredient or a mixture of active principle ingredients.

The semisolid form can be any formulation intended for topical routes of administration to the skin or its appendages. The semisolid form can be sterile or usually nonsterile. It includes semisolid preparations, such as creams, gels, lotions, and ointments. Generally, there is no need for propelling agents to apply the semi-solid form onto the skin or its appendages. In general, semisolid dosage forms are complex formulations having complex structural elements or excipients. Often they are composed of at least two phases (oil and water), one of which is a continuous (external) phase, and the other of which is a dispersed (internal) phase, such as emulsions. The active ingredient is often dissolved in one phase, although occasionally the drug is not fully soluble in the system and is dispersed in one or both phases, thus creating a three-phase system. The physical properties of the dosage form depend upon various factors, including the size of the dispersed particles, the interfacial tension between the phases, the partition coefficient of the active ingredient between the phases, and the product rheology. These factors combine to determine the release characteristics of the drug, as well as other characteristics, such as viscosity.

According to an embodiment, the sample semisolid form placed in the donor compartments of the multi-well microtitre plate in step a) is different from a well to another. According to this embodiment, at least two semisolid forms are each placed preferably in at least two different donor compartments of the multi-well microtitre donor plate, at least one is a sample semisolid form and the other one is a reference sample semisolid form. Preferably, according to this embodiment, the active ingredient present in the semisolid forms is the same (i.e. in terms of quality and quantity) from a well to another.

According to another embodiment, the active principle ingredient contained in the sample semisolid form placed in the donor compartments of the multi-well microtitre plate in step a) is different from a well to another. According to this embodiment, at least two active principle ingredients are preferably placed respectively in at least two donor compartments of the multi-well microtitre plate, at least one is a sample active principle ingredient and the other one is a reference active principle ingredient. Preferably, according to this embodiment, the sample semisolid formulation is the same (i.e. in terms of quality and quantity) from a well to another.

The amount of a semisolid form to be placed can vary in a large extent and depends on the volumes of the donor compartments (or wells) and the surface of the membrane. The amount of a semisolid form to be placed is preferably at least the amount sufficient to cover the overall surface of the membrane. The amount of a semisolid form to be placed of step a) is preferably from 50 to 250mg.

According to the method of the invention, the semisolid form is applied in the donor compartment, the API diffuses through the membrane and is released into the acceptor medium. The membrane is preferably a synthetic membrane. The membrane is preferably selected as to provide an inert holding surface for the test formulation, but not a barrier, so that the API release would reflect the vehicle properties and not the membrane rate-limiting properties. The membrane is preferably selected to have no interaction, physical or chemical, with the semisolid form. Preferably, the nature of the membrane is selected by one skill in the art as to have the less resistant to API diffusion and consequently enhance sensitiveness of the method. According to a preferred embodiment, the membrane is a filter, more preferably a microporous filter. The nature of the membrane is preferably selected in the group consisting of: Polypropylene, Polyethersulfone (PES), hydrophilic or hydrophobic polyvinylidenefluoride (PVDF), Mixed cellulose esters (MCE), hydrophilic or hydrophobic polytetrafluoroethylene, nylon, polycarbonate (PC), polyesters (PET), and polyethyleneterephtalate (PTFE).

According to an embodiment, the thickness of the membrane, preferably the filter, is preferably from 30 to 200 $\mu$m. When the membrane is a micropourous filter, the size of the pores preferably ranges from 0.1 to 4 $\mu$m, preferably from 0.2 to 3 $\mu$m (such as 0.4 $\mu$m).

According to the method of the invention, a 'sandwich' is formed at step c) from a multi-well microtitre donor plate and a multi-well microtitre acceptor plate, also called herein a composite multi-well microtitre plate, such that each formed composite well is divided into two compartments (the donor compartments and the acceptor compartments), separated by the membrane. The number of the wells generally ranges from 6 to 96, preferably from 12 to 48, or is preferably 24.

According to an embodiment, the multi-well microtitre donor plate comprises a hole near each donor compartment so that said hole allows collecting samples or aliquots from the acceptor compartment.

Such composite multi-well microtitre plates are commercially available, for instance they are sold by the Corning® Company, and more specifically the name of the product is HTS Transwell® system. For instance, the HTS Transwell-24 System has an array of 24 wells with permeable inserts connected by a rigid tray that enables all 24 Transwell inserts to be handled as a single unit. The individually packaged product consists of two individually wrapped HTS Transwell-24 units loaded into open reservoirs and includes two 24 well plates. The membrane can be for instance either polycarbonate (PC) or polyester (PET), the pore size of the membrane (microporous filter) is more specifically 0.4 $\mu$m.

The initial acceptor medium used in the present invention should allow a diffusion for the API released from the semisolid form. The initial acceptor medium is preferably an aqueous or hydroalcoholic medium. More specifically, appropriate acceptor medium is aqueous buffer for water soluble API or a hydro-alcoholic medium for sparingly water soluble API. The acceptor medium is selected by one skill in the art in order to have solubility of the active ingredient in the medium. The volume to be placed in the acceptor compartment is such that membrane in the bottom of the donor compartment is into contact with the initial acceptor medium.

More specifically, the initial acceptor medium is placed in the acceptor compartments only. Said medium or a part thereof is not placed in the donor compartment and is therefore not used to initiate dissolution of the API, since the aim of the method of the invention is to determine the release rate of the API that reflects solubility and/or particle size of the API and rheological properties of the dosage form.

According to a particular embodiment, the time period for collecting a sample of the acceptor medium in the acceptor compartment, according to step d), must not exceed the time when more than 30% of the total amount of the API applied is released into the medium, at the end of the experiment. This particular embodiment should avoid receptor back diffusion into donor compartment.

According to an embodiment, according to step d) of the method of the invention, a multiple sampling times (at least 2, 3, 4, 5, or 6 times) over an appropriate time period is carried out, as to generate an adequate release profile and to determine thereafter the API release rate. A 6-hour study period with not less than five samples, i.e., at 30 minutes, 1, 2, 4 and 6 hours is preferred. The sampling times may have to be varied depending on the semisolid form. An aliquot (i.e. a sample) of the acceptor medium is removed at each sampling interval, and preferably replaced with fresh aliquot (i.e., initial acceptor medium), so that the lower surface of the membrane remains in contact with the acceptor medium over the experimental time period. According to an embodiment, sample collection can be automated.

According to an embodiment, sample collection is carried out with a pipette or needle. Automatic pipetting can thus be performed.

The composite multi-well microtitre plate is preferably occluded by a lid, to prevent solvent evaporation and/or compositional changes of formulations.

Aliquots or samples removed from the acceptor compartments are analyzed for API content according to step e) of the method of the invention by high pressure liquid chromatography (HPLC) or any other analytical methodology.

Measuring according to step e) of the invention is preferably carried out as follows: A plot of the amount of released API per unit area ($\mu$g/cm$^2$) against the square root of time (h$^{1/2}$) yields a straight line, the slope of which represents the release rate. Measuring according to step e) can be automated.

This release rate measure is formulation-specific and can be used to monitor product quality. The release rate of the biobatch or currently manufactured batch should be compared with the release rate of the product prepared after a change. Accordingly, the obtained release rates are generally compared with other release rates (obtained previously or obtained in other wells on the same experimental run ot the same multi-well microtitre plate) as to determine whether the different semisolid forms or the different active principle ingredients have an impact or not on the obtained release rates of APIs. More specifically, when two semisolid forms are placed in at least two different donor compartments of the multi-well microtitre plate, where at least one is a sample semisolid form and the other one is a reference sample semisolid form, the obtained release rates of both compartments are compared with each other as to determine whether the sample semisolid form is different from the reference semisolid form.

An insteresting aspect of the invention is that the method can be automated and computerized.

Other aspects and advantages of the present invention will become apparent upon consideration of the following examples, which must be regarded as illustrative and nonrestrictive.

## EXAMPLES

### Materials and Methods

HTS 24 wells micro-plates: HTS Transwell® -24 Well Permeable Support (Corning®), with the following characteristics:

- Polycarbonate or Polyester(PET) - 0.4$\mu$m pore size - membranes sealed to each well
- 0.33cm$^2$ area/well
- 24-well individual reservoir (or acceptor) plate
- 24-well individual receiver (or donor) plate with a hole for sample collection
- a lid to prevent evaporation of volatile excipients

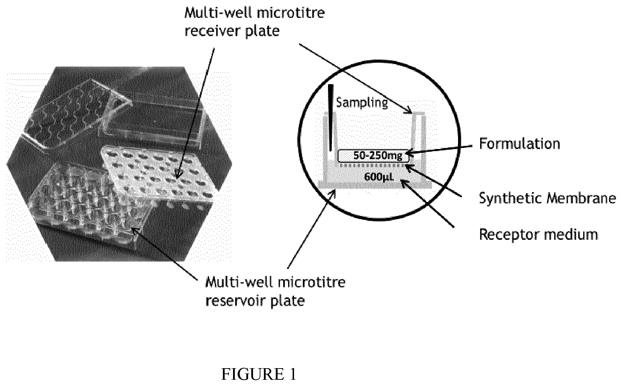

This is illustrated by figure 1.

Vertical glass Franz diffusion cells system

### Results

### - HTS 24 wells micro-plates used for IVRT studies

The aim of this study was to qualify the microplates and assess their ability for the evaluation of the release of an API from a semi-solid dosage form.

Dosage strength was followed with 0.1 to 0.75% (w/w) metronidazole creams by using a composite 24 wells micro-plate according to the invention.

0.75% (w/w) metronidazole gel was used to assess the impact of the viscosity on the release rate of API, by implementing the method according to the invention. Viscosity was modified by adding carbopol 980 (0.2 to 0.9% by weight).

Also, the amount of applied formulations was studied by applying from 50, 75, 125 and 250mg of 0.75% (w/w) metronidazole cream.

Repeatability: N=6 and Slope CV <10%. (CV means Coefficient Variation)

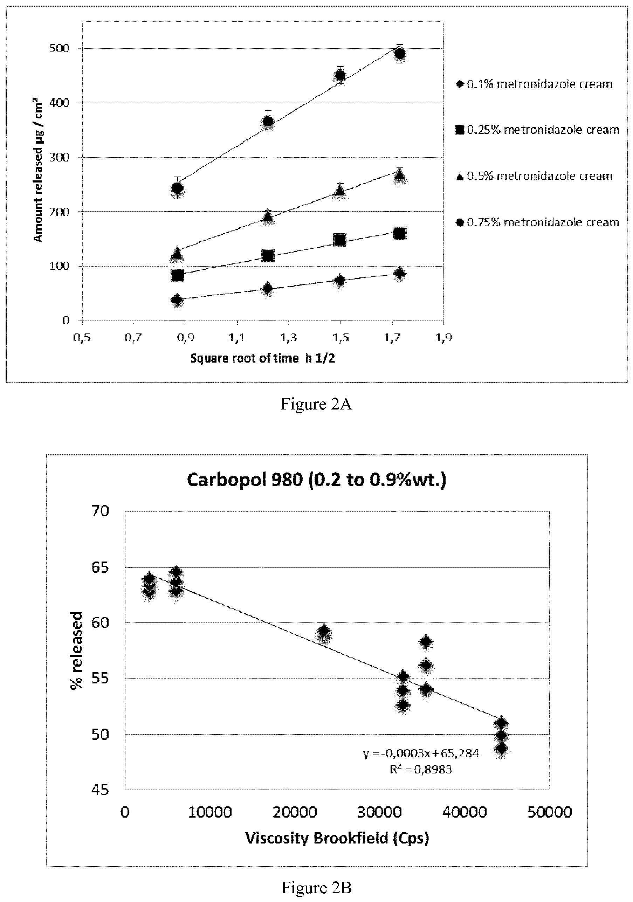

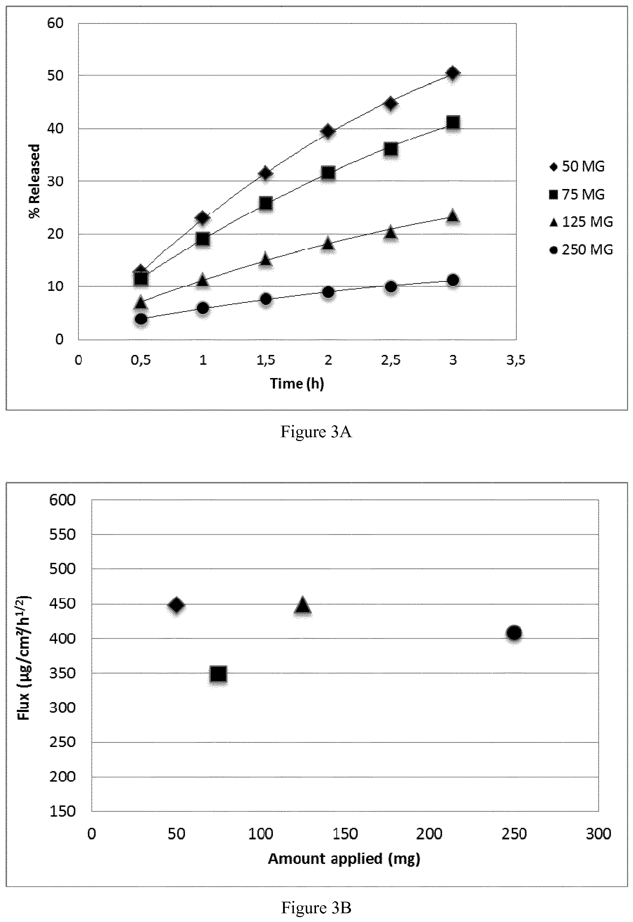

The results are given Figures 2 and 3.

Figure 2A shows that the method according to the invention is highly sensitive to dosage strength.

Figure 2B shows the impact of the viscosity on the release.

Figure 3A and 3B show that the amount of applied formulations impacts the release and not the flux.

In view of these results, microplates can be used for API release studies from semi-solid dosage forms.

**- Comparative study: Franz cell versus micro-plates**

Comparison of the release profile of Metronidazole at 0.5 and 0.75% (w/w) from a cream.

| DIFFUSION CELL SYSTEM | Franz cells | Micro-plates 24 wells |
|---|---|---|
| Synthetic membrane | Polycarbonate | |
| Receptor medium volume (mL) | $\approx 2.5$ | 0.6 |
| Receptor medium composition | Water | |
| Amount of formulation applied (mg) | 750 | 125 |
| Amount of formulation/unit surface area (mg/cm$^2$) | 375 | 380 |
| Number of samples | 6 | 6 |
| Sampling time points | 6 | 6 |
| Study period (h) | 3 | 3 |

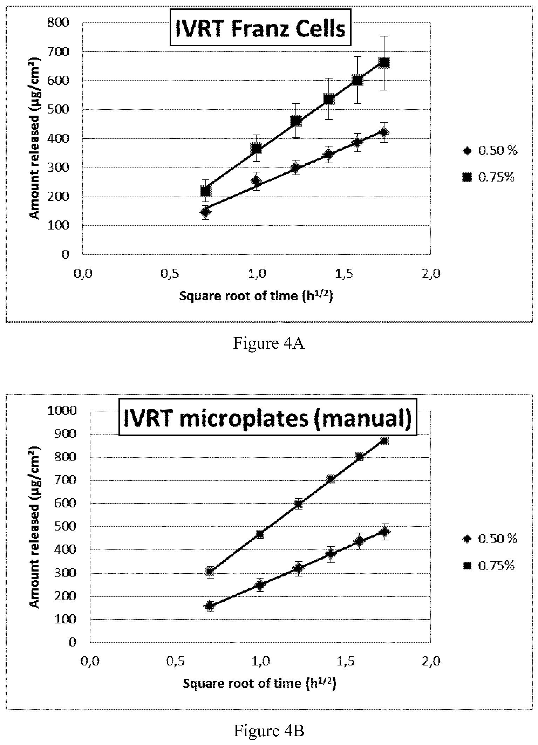

The amount of formulation per unit surface area, considered as important for the comparison study, was identical for the two systems, to get relevant results The results are shown on figure 4A and 4B.
Ranking of formulations is identical between the two systems.
Micro-plates are slightly more sensitive to dosage strength vs. Franz cells. Micro-plates can thus be an alternative to Franz cells for API release studies from semi-solid dosage forms.

**- IVRT using Franz diffusion cells (as recommended by SUPAC Guidelines) versus IVRT using microplates**

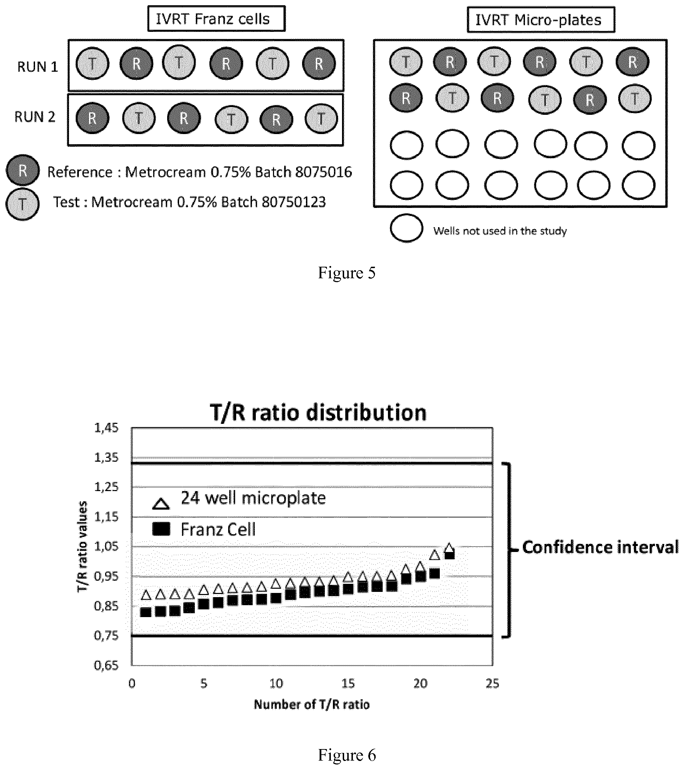

2 batches of commercial 0.75% Metrocream® were assigned to the Franz diffusion cells and micro-plates as described by Figure 5. The experimental description shown on Figure 5 was followed.
At the end of the 2 comparative release studies, the non-parametric statistical method related to Wilcoxon Rank Sum/ Mann-Whitney rank test was applied to the slopes from Franz cells study and Micro-plate study, according to SUPAC Guidelines. The results are shown on Figure 6 (T/R are calculated as defined by SUPAC-SS).
The T/R ratios distribution of the slopes falls within the limits of 75 to 133.33% (90% of Confidence Interval according to the statistical method) and the T/R distribution profile are quite similar between the two systems.
This example shows that micro-plates is an alternative to Franz cells for comparative IVRT studies.

**- Automation of the 24 wells micro-plate**

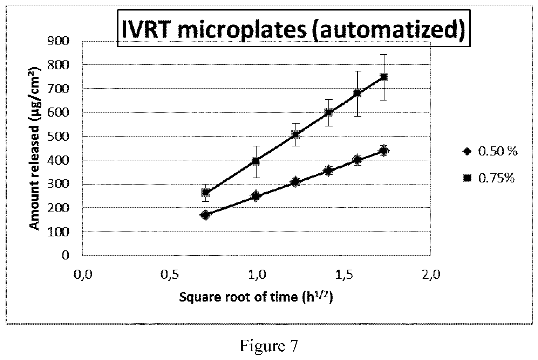

Assess the automation of the methodology used for IVRT studies with micro-plates using a TECAN® robot.
0.5 and 0.75% (w/w) Metronidazole creams were used.
The results are shown Figure 7.
Same ranking is obtained either by using automated (figure 7) or manual (figure 4B) microplates

**Claims**

**1.** A method for determining *in vitro* release rate of at least one active principle ingredient from at least one semisolid form intended for topical routes of administration to skin or its appendages, using a composite multi-well microtitre plate, wherein each well comprises a donor compartment and a corresponding acceptor compartment adapted to receive the donor compartment, the bottom of the donor compartment is made of a porous membrane, the method comprising:

a. Placing an aliquot of a semisolid form comprising at least one active principle ingredient in at least one donor compartment of the multi-well microtitre plate;
b. Placing an initial acceptor medium in the corresponding acceptor compartment(s);
c. Inserting the donor compartment in the corresponding acceptor compartment, so that the membrane in the bottom of the donor compartment is brought into contact with the initial acceptor medium;
d. Collecting at at least two successive time intervals a sample of the acceptor medium in the acceptor compartment;

e. Measuring the release rate of said active principle ingredient in each collected sample,

wherein said at least one semisolid form is a cream, gel, lotion, or ointment.

2. The method of claim 1, wherein the sample semisolid form placed in the donor compartments of the multi-well microtitre plate in step a) is different from one well to another.

3. The method of claim 1 or 2, wherein the membrane is a synthetic membrane.

4. The method of anyone of claims 1-3, wherein the membrane is a filter, more preferably a microporous filter.

5. The method of anyone of claims 1-4, wherein the membrane is selected from the group consisting of: Polypropylene, Polyethersulfone (PES), hydrophilic or hydrophobic polyvinylidenefluoride (PVDF), Mixed cellulose esters (MCE), hydrophilic or hydrophobic polytetrafluoroethylene, nylon, polycarbonate (PC), polyesters (PET), and polyethyleneterephtalate (PTFE).

**Patentansprüche**

1. Ein Verfahren zur Bestimmung der *in vitro* Freisetzungsrate von mindestens einem Wirkstoff aus mindestens einer halbfesten Form, die für topische Verabreichungswege an Haut oder deren Anhänge vorgesehen ist, unter Verwendung einer zusammengesetzten Multi-Well-Mikrotiterplatte, wobei jedes Well ein Donorkompartiment umfasst und ein entsprechendes Akzeptorkompartiment, das zur Aufnahme des Donorkompartiment angepasst ist, der Boden des Donorkompartiment besteht aus einer porösen Membran, wobei das Verfahren umfasst:

a. Platzieren eines Aliquots einer halbfesten Form umfassend mindestens einen Wirkstoff in mindestens einem Donorkompartiment der Multi-Well-Mikrotiterplatte;

b. Platzieren eines initialen Akzeptormediums in das/die entsprechende/ entsprechenden Akzeptorkompartiment(e);

c. Einsetzen des Donorkompartiments in das entsprechende Akzeptorkompartiment, so dass die Membran im Boden des Donorkompartiments mit dem initialen Akzeptormedium in Kontakt gebracht wird;

d. Sammeln einer Probe des Akzeptormediums an mindestens zwei aufeinanderfolgenden Zeitintervallen in dem Akzeptorkompartiment;

e. Messen der Freisetzungsrate des Wirkstoffs in jeder gesammelten Probe,

wobei die mindestens eine halbfeste Form eine Creme, ein Gel, eine Lotion oder eine Salbe ist.

2. Das Verfahren nach Anspruch 1, wobei sich die Probe halbfester Form platziert in die Donorkompartimente der Multi-Well-Mikrotiterplatte in Schritt a) von einem Well zum anderen unterscheidet.

3. Das Verfahren nach Anspruch 1 oder 2, wobei die Membran eine synthetische Membran ist.

4. Das Verfahren nach einem der Ansprüche 1-3, wobei die Membran ein Filter ist, vorzugsweise ein mikroporöser Filter.

5. Das Verfahren nach einem der Ansprüche 1-4, wobei die Membran ausgewählt ist aus der Gruppe bestehend aus: Polypropylen, Polyethersulfon (PES), hydrophilem oder hydrophobem Polyvinylidenfluorid (PVDF), gemischten Celluloseestern (MCE), hydrophilem oder hydrophobem Polytetrafluorethylen, Nylon, Polycarbonat (PC), Polyestern (PET) und Polyethylenterephtalat (PTFE).

**Revendications**

1. Méthode de détermination *in vitro* du taux de libération d'au moins un principe actif à partir d'au moins une forme semi-solide destinée aux voies d'administration topiques sur la peau ou ses annexes, utilisant une plaque de micro-titration multi-puits composite, dans laquelle chaque puits comprend un compartiment donneur et un compartiment accepteur correspondant adapté pour recevoir le compartiment donneur, le fond du compartiment donneur est constitué d'une membrane poreuse, la méthode comprenant :

a- Placer un aliquot d'une forme semi-solide comprenant au moins un principe actif dans au moins un compartiment donneur de la plaque de micro-titration multi-puits ;

b- Placer un milieu accepteur initial dans le ou les compartiment(s) accepteur(s) correspondant(s) ;

c- Insérer le compartiment donneur dans le compartiment accepteur correspondant, de sorte que la membrane du fond du compartiment donneur soit mise en contact avec le milieu accepteur initial ;

d- Collecter à au moins deux intervalles de temps successives un échantillon du milieu accepteur dans le compartiment accepteur ;

e- Mesurer le taux de libération dudit principe actif dans chaque échantillon collecté,

dans laquelle ladite au moins une forme semi-solide est une crème, un gel, une lotion, ou une pommade.

2. Méthode selon la revendication 1, dans laquelle l'échantillon de forme semi-solide placé dans les compartiments donneurs de la plaque de micro-titration multi-puits de l'étape a) est différent d'un puits à l'autre.

3. Méthode selon la revendication 1 ou 2, dans laquelle la membrane est une membrane synthétique.

4. Méthode selon l'une quelconque des revendications 1 à 3, dans laquelle la membrane est un filtre, plus particulièrement un filtre microporeux.

5. Méthode selon l'une quelconque des revendications 1 à 4, dans laquelle la membrane est choisie parmi le groupe constitué par : Polypropylène, Polyéthersulfone (PES), Polyfluorure de vinylidène (PVDF) hydrophile ou hydrophobe, mélanges d'esters de cellulose (MCE), Polytétrafluoroéthylène hydrophile ou hydrophobe, nylon, polycarbonate (PC), polyesters (PET), et Polyéthylène téréphtalate (PTFE).

Multi-well microtitre
receiver plate

Sampling

Formulation

50-250mg

600μL

Synthetic Membrane

Receptor medium

Multi-well microtitre
reservoir plate

FIGURE 1

Figure 2A

Figure 2B

Figure 3A

Figure 3B

Figure 4A

Figure 4B

Figure 5

Figure 6

Figure 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005095950 A